# EUROPÄISCHE PATENTANMELDUNG

(11) **EP 1 844 779 A2**
(43) Veröffentlichungstag der Anmeldung: **17.10.2007**
(21) Anmeldenummer: 07005797.1
(22) Anmeldetag: 21.03.2007
(51) Int. Cl.: A61K 31/48, A61P 25/00

(54) **Tergurid/Protergurid zur Behandlung von chronischen Schmerzen (z.B. Migräne)**

(30) Priorität: 21.03.2006 DE 102006013307
(71) Anmelder: ErgoNex Pharma GmbH, 9050 Appenzell (CH)
(72) Erfinder: Ludwig, Georg, 9492 Eschen (LI); Horowski, Reinhard, Dr., 13353 Berlin (DE); Bliesath, Harald, Dr., 78473 Allensbach (DE); Reiter, Rudolf, Dr., 6940 Appenzell (CH)
(74) Vertreter: Arth, Hans-Lothar

(57) **Zusammenfassung**

Die vorliegende Erfindung betrifft die Verwendung von Tergurid und Protergurid zur Prophylaxe und/oder Behandlung von chronischen Schmerzzuständen wie z.B. Rückenschmerzen, Kopfschmerzen, Rheumatische Schmerzen, Neuralgien sowie pharmazeutische Zusammensetzungen enthaltend Tergurid und/oder Protergurid optional zusammen mit Opiat Analgetikum.

## Beschreibung

Die vorliegende Erfindung betrifft die Verwendung von Tergurid und Protergurid zur Prophylaxe und / oder Behandlung chronischer Schmerzzustände sowie pharmazeutische Zusammensetzungen enthaltend Tergurid und/oder Protergurid optional zusammen mit einem Opiat Analgetikum.

Der akute Schmerz dient als Alarmsignal für den Organismus und führt zu raschen Verhütungs- und Schutzreaktionen. Intensive akute Schmerzreize können innerhalb kurzer Zeit anhaltende funktionelle und strukturelle Veränderungen bewirken, welche die Reizweiterleitung und -verarbeitung nachhaltig verändern. Die Konsequenz sind chronische Schmerzen ohne einen erkennbaren physiologischen Nutzen.

Dauert ein Schmerzgeschehen länger als drei bis sechs Monate an, spricht man von chronischem Schmerz. Ursache hierfür können unheilbare Erkrankungen wie zum Beispiel bösartige Tumoren oder rheumatische Erkrankungen sein. Oft ist jedoch kein Zusammenhang mehr zwischen dem Schmerz und der Schädigung bzw. der Erkrankung, die einst den Schmerz auslöste, erkennbar oder die ursprüngliche Schädigung kann nicht mehr behoben werden. Zudem können viele Umwelteinflüsse wie Stress oder Wetteränderungen die Schmerzen triggern oder verstärken. Oft sind verschiedene Schmerzformen bei einer chronischen Schmerzsymptomatik vereint.

Als die häufigsten chronischen Schmerzformen werden Rückenschmerzen (u.a. nach Bandscheibenvorfall, Nervenwurzelkompressionssyndrom), Kopfschmerzen (u.a. Migräne, Spannungskopfschmerz, Clusterkopfschmerz), Rheumatische Schmerzen (u.a. Arthritis, Fibromyalgie), Neuralgien (u.a. Trigeminusneuralgie, Gürtelrose), Tumorschmerzen (u.a. Hirntumore, Knochenmetastasen), degenerative Schmerzen (u.a. Osteoporose, Arthrose) und Phantomschmerzen (u.a. nach Amputation, Plexusabriss) genannt.

Chronische Schmerzen dauern oft über viele Jahre oder Jahrzehnte an. Patienten, die unter chronischen Schmerzen leiden, entwickeln häufig emotionale Probleme. Viele Schmerzpatienten leiden unter Lust und Antriebslosigkeit; sie sind hoffnungslos und verzweifelt, klagen über Angstgefühle und Depressivität, empfinden sich in ihrem Selbstwertgefühl eingeschränkt. Solche psychischen Symptome sind ebenso Warnzeichen für eine Chronifizierung wie allgemeine, unspezifische körperliche Beschwerden, etwa Darmprobleme (Durchfall bzw. Verstopfung), Reizblase, Schwindel, Atemnot, Herzklopfen oder Engegefühl im Brustkorb.

Verschiedene Mechanismen im peripheren und zentralen Nervensystem sind an der Entstehung von chronischen Schmerzen beteiligt: Die Sensibilisierung von Schmerzfasern und ihre lokale Übererregbarkeit sind wesentliche pathogenetische Mechanismen, die im Bereich der peripheren Schmerzwahrnehmung bei der Entwicklung von chronischen Schmerzzuständen eine Rolle spielen. Weitere Pathomechanismen umfassen die länger andauernde Verstärkung von Schmerzsignalen und eine Rekrutierung von üblicherweise stummen Nervenfasern im Bereich des Rückenmarks, die zu einer vergrößerten räumlichen Ausdehnung der Schmerzempfindung führen. Im Gehirn kommt es schließlich durch die vermehrt aus der Peripherie eintreffenden Schmerzpotenziale zu Veränderungen in der Signalweiterleitung im Sinne einer Verstärkung der Schmerzwahrnehmung und einer längerfristigen Veränderung der Schmerzverarbeitung.

Intensive Schmerzreize können bereits nach wenigen Minuten zu anhaltenden strukturellen und funktionellen Veränderungen führen, welche die Weiterleitung und Verarbeitung von Schmerzreizen intensivieren. Diese Vorgänge gleichen zellulären Abläufen, wie sie bei allen komplexeren neuronalen Lernvorgängen beobachtet werden können, dementsprechend wird in Analogie auch von einem Schmerzgedächtnis gesprochen. Der Begriff Schmerzgedächtnis beinhaltet dabei die Fähigkeit des Nervensystems, eine Erinnerungsspur für eine eingetretene schmerzhafte Reizung durch das ganze schmerzverarbeitende System zu legen.

Bestimmten Nervenkernen im Gehirn kommt hierbei eine Schlüsselfunktion in der Schmerzverarbeitung zu. Für aufsteigende Schmerzimpulse aus dem Körper dient der Nucleus accumbens als zentrale Schaltstelle für die Weiterleitung und Interpretation von Schmerzreizen. In ähnlicher Weise kommt dem Nucleus raphe eine Schüsselrolle für die Weiterleitung und Modulierung von absteigenden Schmerzsignalen in den Körper zu.

Für die Behandlung von chronischen Schmerzen ist ein umfangreiches Arsenal von Arzneimitteln verfügbar: Nonsteroidale entzündungshemmende Arzneimittel (NSAIDs) und nicht-Opiat Analgetika sind die am häufigsten zur Schmerzmedikation genutzten Arzneimittel. NSAIDs haben analgetische, entzündungshemmende und antipyretische Eigenschaften und werden zur Behandlung von milden bis moderaten Schmerzen eingesetzt. Andere nicht-Opiat Analgetika wie beispielsweise Aspirin, Paracetamol oder COX-I/II Inhibitoren werden häufig in Kombination mit Opioiden wie Oxycodon und Hydrocodon zur Behandlung von mäßigen bis starken Schmerzen eingesetzt. Opiate wie Morphin, Fentanyl, Methadon, Hydromorphon, Oxycodon, Hydrocodon und Meperidin finden in der Behandlung von starken Schmerzen Anwendung.

Die genannten Vertreter gewährleisten allerdings häufig nur eine teilweise Schmerzlinderung, verlieren nach kurzer Zeit ihre Wirksamkeit und sind oft mit deutlichen Nebenwirkungen verbunden. In diesem Zusammenhang sind insbesondere die NSAIDs sowie Opiate zu nennen. Beide Wirkstoffklassen weisen schwerwiegende Nebenwirkungen auf. Als Nebenwirkungen der Therapie mit NSAIDs sind gastrointestinale Störungen und Geschwüre, Nierenschäden und Hypersensitivitätsreaktionen beschrieben. Als Nachteile von zentral wirkenden Opiaten, wie beispielsweise Morphin und Oxycodon sind neben gastrointestinalen Nebenwirkungen wie Obstipation eine Reihe von zentralnervös mediierten Nebenwirkungen wie Schläfrigkeit, Übelkeit, Verwirrung, Atmungsdepression, Wirksamkeitsverlust und körperliche oder physische Abhängigkeit zu nennen.

Es besteht deshalb nach wie vor ein Bedarf an besser wirksamen analgetischen Wirkstoffen mit Wirksamkeit über ein breites Spektrum von Schmerzzuständen, welche zudem minimale oder keine Nebenwirkungen haben und welche nicht zur Toleranzentwicklung oder zur körperlichen oder physischen Abhängigkeit führen.

Aufgabe der vorliegenden Erfindung ist es somit, ein Arzneimittel zur Behandlung von Fibromyalgie und chronischen Schmerzzuständen bereitzustellen, welches die Symptome effektiv bekämpft und deutlich weniger Nebenwirkungen aufweist, als die aus dem Stand der Technik bekannten Arzneimittel.

Diese Aufgabe wird durch die technische Lehre der unabhängigen Ansprüche gelöst. Weitere vorteilhafte Ausgestaltungen, Aspekte und Details der Erfindung ergeben sich aus den abhängigen Ansprüchen, der Beschreibung und den Beispielen.

Die vorliegende Erfindung offenbart die Verwendung von Tergurid oder Protergurid oder einer Kombination aus beidem zur Herstellung von Arzneimitteln zur Prophylaxe und Behandlung von chronischen Schmerzen und chronischen Schmerzzuständen.

Überraschend wurde gefunden, dass zusätzlich zu den bekannten Effekten der erfindungsgemäßen Wirkstoffe bei M. Parkinson, Restless Legs Syndrome und Hyperprolactinämie Tergurid und Protergurid ausgesprochen wirksam in der Behandlung von chronischen Schmerzzuständen sind. Die Wirkung erfolgt in einem gut verträglichen Dosisbereich ohne oder mit nur geringen, transienten Nebenwirkungen wie Nausea, Emesis und orthostatische Störungen. Zudem ist die Abwesenheit von negativen Effekten der erfindungsgemäßen Substanzen auf Schlafprofil und -qualität zu nennen, die eine nebenwirkungsarme Behandlung von Schmerzzuständen möglich macht.

Insbesondere bei persistierenden muskuloskeletalen Schmerzen und persistierenden viszeralen Schmerzen haben sich Tergurid und Protergurid sehr effektiv erwiesen.

Somit sind die beiden Verbindungen erfindungsgemäß zur Behandlung von persistierenten Rückenschmerzen, Rückenschmerzen beispielsweise nach Bandscheibenvorfall oder Nervenwurzelkompressionssyndrom, persistierenten Nackenschmerzen, persistierenten Schulterschmerzen, persistierenten Gelenkschmerzen, rheumatische Schmerzen, Arthritis, Fibromyalgie und Chronic Fatigue Syndrom geeignet, wobei Fibromyalgie insbesondere bevorzugt ist.

Unter Chronic Fatigue Syndrom (CFS), welches auch als Chronisches Müdigkeitssyndrom oder Chronisches Erschöpfungssyndrom bekannt ist, versteht man eine lähmende geistige und körperliche Erschöpfung bzw. Erschöpfbarkeit und weitere, individuell unterschiedliche Symptome der Erschöpfung. Die Erschöpfung muss mindestens 6 Monate andauern, um sie als Chronic Fatigue Syndrom bezeichnen zu können und führt zu einer schwerwiegenden Leistungsminderung gegenüber dem früher Gewohnten, was ein normales Leben für den Erkrankten unmöglich macht.

Ferner eignen sich Tergurid und Protergurid sowie Kombinationen aus beidem sehr gut zur Prophylaxe und Behandlung von Kopfschmerzen und Migräne. Tergurid oder Protergurid in geringen Dosen prophylaktisch oder bei den ersten Anzeichen von Kopfschmerzen und Migräne eingesetzt, lindert diese Beschwerden deutlich und teilweise innerhalb weniger Stunden.

Ferner sind die Substanzen Tergurid und Protergurid sehr gut zur Prophylaxe und Behandlung von Schmerzen beim prämenstruellen Syndrom, Mastalgie, von Bauchschmerzen beim Reizdarm sowie von Schmerzen beim Carzinoid Syndrom geeignet. Weitere gut mit Tergurid und/oder Protergurid zu behandelnde Schmerztypen sind degenerative Schmerzen, Osteoporose, Arthrose als auch Phantomschmerzen beispielsweise nach Amputation oder Plexusabriss.

Erfindungsgemäß können mit den beiden Wirkstoffen auch Schmerzen bzw. Schmerzzustände bei Neuralgien, Trigeminusneuralgie, Gürtelrose, postherpetischen Neuralgien sowie neuropathische Schmerzen und Tumor-assoziierte Schmerzen adressiert werden. Die Tumorschmerzen werden insbesondere durch Hirntumore oder Knochenmetastasen verursacht.

Ein weiterer Aspekt der vorliegenden Erfindung ist auf eine Kombination aus Tergurid oder Protergurid oder Tergurid und Protergurid mit einem Opiat Analgetikum sowie auf die Verwendung dieser Kombination zur Prophylaxe und Behandlung der hierin genannten Indikationen gerichtet.

Es hat sich gezeigt, dass eine Kombination aus Tergurid und mindestens einem Opiat Analgetikum oder eine Kombination aus Protergurid und mindestens einem Opiat Analgetikum oder eine Kombination aus Tergurid und Protergurid und mindestens einem Opiat Analgetikum eine bessere Wirkung als die jeweiligen Einzelkomponenten hat, sei es dass die unerwünschten Nebenwirkungen des mindestens einen Opiat Analgetikums reduziert oder die Aktivität des mindestens einen Opiat Analgetikums gesteigert worden ist.

Als Opiat Analgetika können insbesondere folgende Verbindungen genannt werden: Dihydrocodein, Tramadol, Morphin, Morphin-Sulfat, Oxycodon, Methadon, Hydromorphon, Buprenorphin sowie Fentanyl.

Die erfindungsgemäßen Anwendungen sind für eine Daueranwendung geeignet, da keine körperliche oder psychische Abhängigkeit eintrat und auch bei Verabreichung über mehrere Monate kein Wirksamkeitsverlust beobachtet wurde. Darüber hinaus konnte auch gezeigt werden, dass Tergurid als auch Protergurid auch in Fällen, in denen es nach Abklingen der Schmerzsymptomatik abgesetzt wurde, bei Neueintritt der Symptomatik wieder mit vergleichbarer Wirksamkeit eingesetzt werden konnte.

Die erfindungsgemäßen Substanzen zeichnen sich durch eine breite Wirkung auf die dopaminerge, serotoninerge und noradrenerge Neurotransmittersysteme aus. In den Wirkstoffen sind unterschiedliche Wirkprinzipien mit Relevanz für verschiedene zentralnervöse Schmerzleitungssysteme in demselben Molekül zusammengefasst. Es wird vermutet, dass gerade diese Kombination von Wirkqualitäten in den erfindungsgemäßen Substanzen von entscheidender Bedeutung für die hohe, therapeutische Wirksamkeit ist. Zugleich begründet diese Kombination eine positive Wirkung auf die Stimmung, die kognitiven Leistungen und die alltäglichen Aktivitäten von Patienten. Eine überraschende positive Wirkung auf das Gesamtbefinden von Patienten, eine Verbesserung der Schlafqualität sowie die gute Verträglichkeit der Wirkstoffe unterstützen die gute Compliance von Patienten und tragen entscheidend zum Therapieerfolg der erfindungsgemäßen Wirkstoffe bei.

Ein weiterer Aspekt der vorliegenden Erfindung betrifft pharmazeutische Zusammensetzungen, welche neben Tergurid oder Protergurid oder einer Kombination aus Tergurid und Protergurid bzw. deren pharmakologisch verträglichen Salze mindestens einen pharmakologisch verträglichen Träger, Hilfsstoff und/oder Lösungsmittel enthalten.

Des weiteren ist bevorzugt wenn die pharmazeutische Zusammensetzung zudem noch mindestens ein Opiat Analgetikum enthält. Dieses mindestens eine Opiat Analgetikum kann zusammen mit Tergurid und/oder Protergurid in einer einzigen galenischen Formulierung enthalten sein oder als eine zweite galenische Formulierung vorliegen, welche unabhängig von der Formulierung enthaltend Tergurid und/oder Protergurid appliziert werden kann. Das Vorliegen von zwei Formulierungen in der pharmazeutischen Zusammensetzungen ist zudem bevorzugt, da so eine bestimmte Konzentration von Tergurid und/oder Protergurid zum Opiat Analgetikum besser eingestellt bzw. verändert oder das Opiat Analgetikum gänzlich abgesetzt werden kann.

Diese pharmazeutischen Zusammensetzung enthalten vorzugsweise Tergurid im Dosisbereich von 0,1 - 3,0 mg pro pharmazeutischer Formulierung oder Protergurid im Dosisbereich von 0,002 - 0,5 mg pro pharmazeutischer Formulierung. Enthält die pharmazeutische Formulierung Tergurid als auch Protergurid so sind weiterhin die Dosisbereiche von 0,1 - 3,0 mg für Tergurid und 0,002 - 0,5 mg für Protergurid bevorzugt.

Die pharmazeutischen Zusammensetzung werden bevorzugt in Form von Pillen, Tabletten, magensaftresistenten Tabletten, Filmtabletten, Schichttabletten, Retard-Oralia, Dragees, Zäpfchen, Gelen, Salben, Sirup, Inhalationspulvern, Granulaten, Suppositorien, Emulsionen, Dispersionen, Mikrokapseln, Mikroformulierungen, Nanoformulierungen, liposomalen Formulierungen, Kapseln, magensaftresistenten Kapseln, Pulver, Inhalationspulvern, mikrokristallinen Formulierungen, Inhalationssprays, Puder, Tropfen, Nasentropfen, Nasensprays, Aerosolen, Ampullen, Lösungen, Säfte, Suspensionen, Emulsionen, Infusionslösungen oder Injektionslösungen bereitgestellt. Bevorzugt sind Kapseln, Dragees, magensaftresistente Formulierungen, Säfte, Suspensionen, Suppositorien, Lösungen, Injektionen und Granulate.

Die pharmazeutischen Zusammensetzungen sind bevorzugt zur Inhalation oder zur intravenösen, intraperitonealen, intramuskulären, intravaginalen, intrabuccalen, perkutanen, subkutanen, mucokutanen, oralen, peroralen, lumbalen, rektalen, transdermalen, topikalen, intradermalen, intragastralen oder intrakutanen Applikation geeignet.

Als pharmakologisch verträgliche Träger können beispielsweise Lactose, Stärke, Sorbitol, Sucrose, Cellulose, Magnesiumstearat, Dicalciumphosphat, Calciumsulfat, Talk, Mannitol, Ethylalkohol und dergleichen eingesetzt werden. Puder als auch Tabletten können zu 5 bis 95% aus einem derartigen Träger bestehen.

Als Bindemittel können zudem Stärke, Gelatine, natürliche Zucker, sowohl natürliche als auch synthetische Gummis wie beispielsweise Akaziengummi oder Guar-Gummi, Natriumalginat, Carboxymethyl-Cellulose, Polyethylenglycol und Wachse eingesetzt werden. Als Gleitmittel können Borsäure, Natriumbenzoat, Natriumacetat, Natriumchlorid, und dergleichen dienen.

Ferner können den pharmazeutischen Zusammensetzungen noch Sprengmittel, Farbstoffe, Geschmacksstoffe und/oder Bindemittel zugesetzt werden.

Flüssige Formulierungen umfassen Lösungen, Suspensionen, Sprays und Emulsionen. Beispielsweise Injektionslösungen auf Wasserbasis oder Wasser-Propylenglycol-Basis für parenterale Injektionen.

Für die Zubereitung von Suppositorien werden bevorzugt niedrigschmelzende Wachse, Fettsäureester und Glyceride eingesetzt.

Kapseln werden beispielsweise aus Methylcellulose, Polyvinylalkohole oder denaturierte Gelatine oder Stärke hergestellt.

Als Sprengmittel können Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, Natrium-Carboxymethylcellulose, mikrokristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Korn, Reis oder Kartoffeln, natürliche Gummis wie Akaziengummi, Gelatine, Tragacanth, Alginsäure, Natriumalginat, Ammonium-Calcium-Alginat, Methylcellulose, Natrium-Carboxymethylcellulose, Hydroxypropyl-methylcellulose, Polyvinylpyrrolidon sowie anorganische Verbindungen wie Magnesium-Aluminum-Silicate zugesetzt werden. Die Bindemittel könne in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol und Aminosäuren wie Leucin eingesetzt werden. Derartige Gleitmittel können in Mengen von 0,05 bis 15 Gew.-% verwendet werden.

### Beispiele

### Beispiel 1: Anwendung von Tergurid bei Fibromyalgie

Eine Frau im Alter von 36 Jahren, welche an Fibromyalgie litt, wurde mit Tergurid behandelt.

Die Patientin klagte über chronische Schmerzen in den Extremitäten sowie Magenschmerzen und permanente Erschöpfung und Müdigkeit. Die Beschwerden bestanden seit mehreren Monaten. Eine Vorbehandlung war bis zum Zeitpunkt der Tergurid-Behandlung nicht durchgeführt worden.

Die Patientin wurde mit einer täglichen Dosis von 3 mg Tergurid behandelt, welches morgens und abends in Einzeldosen von 1.5 mg verabreicht wurde. Die Behandlung erfolgte einschleichend. Über einen Zeitraum von 2 Wochen wurde die Tagesdosis von anfänglich 0,25 mg schrittweise auf 3 mg erhöht.

Die Behandlung wurde über einen Zeitraum von 25 Wochen durchgeführt und wöchentlich wurde der Zustand der Patientin überprüft. Bereits nach 10 Wochen zeigten sich deutliche Verbesserungen insbesondere hinsichtlich der Schmerzen in den Extremitäten und den Bauchschmerzen, die mit einer visuellen Analogskala erfasst wurden.

Nach 17 Wochen waren auch die chronischen Zustände von Müdigkeit und Erschöpfung fast nicht mehr detektierbar und nach dem Empfinden der Patienten war eine normale Leistungsfähigkeit, so wie sie vor der Erkrankung bestanden hatte wiederhergestellt.

### Beispiel 2: Anwendung von Protergurid bei Fibromyalgie

Eine Frau im Alter von 29 Jahren, welche an Fibromyalgie litt, wurde mit Protergurid behandelt.

Die Patientin klagte über Magenprobleme, Bauchschmerzen, Schmerzen im Brustbereich sowie geschwollene Brüste und andauernde Erschöpfung und Müdigkeit. Die Beschwerden bestanden seit ca. einem Jahr. Eine Vorbehandlung war bis zum Zeitpunkt der Protergurid-Behandlung nicht durchgeführt worden.

Die Patientin wurde mit einer täglichen Dosis von 0,5 mg Protergurid behandelt, welches morgens und abends eingenommen wurde. Die Behandlung erfolgte einschleichend. Über einen Zeitraum von 2 Wochen wurde die Tagesdosis von anfänglich 0,05 mg schrittweise auf 0,5 mg erhöht.

Die Behandlung wurde über einen Zeitraum von 33 Wochen durchgeführt und wöchentlich wurde der Zustand der Patientin überprüft. Bereits nach 12 Wochen zeigten sich deutliche Verbesserungen insbesondere hinsichtlich der Schmerzen im Brustbereich. Zudem war ein Rückgang der Schwellung festzustellen.

Nach 20 Wochen waren auch die chronischen Zustände von Müdigkeit und Erschöpfung sowie die Bauchschmerzen weitgehend verschwunden und nach 30 Wochen fühlte sich die Patientin wieder in einer Verfassung, in der sie vor der Erkrankung war.

### Beispiel 3: Anwendung von Tergurid bei Mastalgie / prämenstruellem Syndrom

Eine 34-jährige Patientin, die an prämenstruellem Syndrom mit Mastalgie und Bauchschmerzen litt, wurde mit Tergurid behandelt. Labormedizinische Untersuchungen zeigten, dass die Prolaktinblutspiegel der Frau im Normalbereich lagen. Die Therapie wurde zur Cyclusmitte gestartet. Der Patientin wurde zweimal täglich eine Tablette mit 0,5 mg Tergurid über einen Zeitraum von 3 Monaten verabreicht. Der Schweregrad der Schmerzsymptomatik wurde mit Hilfe einer visuellen Analogskala verfolgt. Unter Therapie nahmen die Schmerzen im Brust- und Bauchbereich ab und die Patientin konnte auf die Einnahme von zusätzlicher Schmerzmedikation verzichten. Die Patientin berichtete zudem ein deutlich verbessertes Allgemeinbefinden und Lust am Leben. Nebenwirkungen wurden nicht beobachtet. In den darauf folgenden Monaten unter Therapie blieb die Patientin beschwerdefrei.

### Beispiel 4: Anwendung von Tergurid zur Migräne Prophylaxe

Ein 65 Jahre alter Patient, der im Mittel 4 mal monatlich unter Migräne litt, wurde mit Tergurid behandelt. Der Patient wurde mit einer täglichen Dosis von 2 mg Tergurid behandelt, welches morgens und abends in Einzeldosen von 1 mg verabreicht wurde. Die Behandlung erfolgte einschleichend. Über einen Zeitraum von 2 Wochen wurde die Tagesdosis von anfänglich 0,25 mg schrittweise auf 2 mg erhöht. Die Behandlung erfolgte über einen Zeitraum von 24 Wochen. Die Häufigkeit, Dauer und Schweregrad der Migräneanfälle wurde mit Hilfe eines Tagebuches erfasst. Innerhalb von 4 Wochen nach Therapiebeginn war eine 40%ige Reduzierung der Häufigkeit von Migräneanfällen nachweisbar, ebenso wie eine Verkürzung der Dauer der Anfälle von kumulativ 12 Tagen vor Therapie auf 8 Tage unter Tergurid. Der Verbrauch von Schmerzmedikation unter Therapie mit Tergurid war um 46% reduziert. Nebenwirkungen der Terguridtherapie wurden nicht beobachtet. Der Patient beschrieb eine Verbesserung seines Allgemeinbefindens.

### Beispiel 5: Diabetische Neuropathie

Bei einer 56 Jahre alte Patientin, die seit 10 Jahren an Type II Diabetes litt, wurde vor ca. 15 Monaten die klinische Diagnose einer diabetischen Neuropathie gestellt.

Die Patientin wurde mit 3 * 50 mg Amitryptilin / Tag behandelt. Zum Untersuchungszeitpunkt klagte die Patientin über Schmerzustände mit Brennen, einschießende oder stechende Schmerzen und unangenehmem Kribbeln vor allem an den Füßen und während der Nacht mit zunehmender Intensität. Die Patientin gab die Schmerzstärke auf der numerischen Ratingskala von 0 = kein und 10 = max mit durchschnittlichen Schmerzwerten mit Zahlen von durchschnittlich 8 - 9 an.

Unter Therapie mit Tergurid (0.5 mg bid) kam es zu einer raschen Verbesserung der Schmerzzustände (durchschnittliche Schmerzwerte von 2 - 3 auf der Ratingskala) und des Allgemeinbefindens der Patientin. Nebenwirkungen der Tergurid-Therapie wurden nicht beobachtet. Anzeichen eines Wirkverlustes unter fortdauernder Behandlung mit Tergurid wurden auch nicht beobachtet.

### Beispiel 6: Post-herpetische Neuralgie

Ein 65-jähriger normalgewichtiger Patient klagte über ein heftiges brennendes Gefühl und Schmerzen im Hautbereich des Brustkorbes. Die Anamnese ergab eine Erkrankung an Gürtelrose, die allerdings erst nach Eruption des Ausschlags und Verschorfung behandelt wurde. Die Behandlung der Schmerzen mit Amitriptylin (3 * = 50 mg/Tag) und nachfolgend mit Gabapentin (Tagesdosis: 1500 mg) führte nur zu einer kurzfristigen Besserung, die innerhalb von 10 Tagen von einer massiven Verschlimmerung der Schmerzen gefolgt war.

Unter Gabe von Tergurid (0.5 mg tid) kam es zu einer raschen und anhaltenden Verbesserung der Schmerzsymptomatik. Nebenwirkungen wurden nicht beobachtet. Nach 3 Monaten wurde im Rahmen eines Auslassversuches die Tagesdosis von Tergurid schrittweise reduziert, wobei eine rasche Zunahme der Schmerzen erfolgte.

### Beispiel 7: Tumorschmerz

Ein 62-jähriger Tumorpatient mit Pankreaskarzinom klagte über Knochenschmerzen, die auf die Ausbildung von Knochenmetastasen zurückgeführt werden könnten. Der Patient wurde mit Ibandronat behandelt. Zusätzlich erfolgte eine Behandlung mit Ibuprofen, Acetaminophen und Desipramin durchgeführt. Die Therapie führte kurzfristig zur Schmerzlinderung, die allerdings innerhalb weniger Wochen stark verschlechterte. Nach Start der Behandlung mit Tergurid (0.5 mg bid) kam es zu einer anhaltenden Verbesserung des Schmerzzustandes und des Wohlbefindens des Patienten. Nebenwirkungen wurden nicht beobachtet.

### Beispiel 8: Bandscheibenvorfall

Seit zwei Jahren litt der Patient (43 Jahren) an einem Bandscheibenvorfall (im unteren Lendenbereich (L5-S1), mit Ausdehnung auf L4-L5). Die chronischen Schmerzen wurden mit Oxycodon in einer anfänglichen Dosierung von 2*10 mg und Dosissteigerung bis 2*40 mg pro Tag behandelt. Die Schmerzintensität wurde unter Therapie stark reduziert. Mit zunehmender Behandlungsdauer traten zunehmend Beschwerden durch Obstipation auf, die schliesslich eine Dosisreduktion von Oxycodon auf 2*20 mg/Tag erforderlich machten. Dies war mit einem Anstieg der subjektiven Schmerzintensität des Patienten verbunden. Unter zusätzlicher Behandlung mit Tergurid (2*0.5 mg/Tag) stellte sich eine nahezu vollständige Schmerzfreiheit des Patienten wieder ein; ebenso verschwanden Symptome der Obstipation vollständig. Die Wirksamkeit der Kombinationstherapie mit Tergurid blieb unter chronischer Therapie erhalten.

### Beispiel 9: Migräne Prophylaxe

Der 47jährige Patient litt an Migräne Kopfschmerzen mit durchschnittlich 4-5 Attacken pro Monat mit einer Länge der Attacken von 3-8 Stunden. Zur akuten Schmerzbehandlung wurde im Anfall Sumatriptan in einer Dosierung von 50 mg oral eingesetzt. Zur Migräneprophylaxe wurde der Patient mit Topiramat in einer Dosierung von 200 mg pro Tag behandelt. Unter Medikation wurde keine Verringerung der Migräne-Attacken beobachtet. Nach 3 Monaten wurde das Medikament wegen Übelkeit und Gewichtsverlust abgesetzt.

Unter Terguridgabe (2*0.25 mg / Tag) verringerte sich die Zahl der Schmerzattacken dramatisch auf eine einzige Schmerzattacke innerhalb von 6 Wochen mit geringer Schmerzintensität, auch das Wohlbefinden des Patienten verbesserte sich innerhalb 2 Wochen nach Beginn der Therapie. Nebenwirkungen wurden nicht beobachtet. Im Rahmen eines Auslassversuches wurde die Behandlung mit Tergurid eingestellt, worauf hin die Migränesymptomatik wieder zurückkehrte.

## Patentansprüche

1. Verwendung von Tergurid oder Protergurid oder einer Kombination aus Tergurid und Protergurid oder deren pharmakologisch verträgliche Salze zur Herstellung eines Arzneimittels zur Prophylaxe und / oder Behandlung von chronischen Schmerzen und chronischen Schmerzzuständen.

2. Verwendung gemäß Anspruch 1 zur Behandlung von persistierenden muskuloskeletalen Schmerzen und persistierenden viszeralen Schmerzen.

3. Verwendung gemäß Anspruch 2 zur Behandlung von persistierenten Rückenschmerzen, persistierenten Nackenschmerzen, persistierenten Schulterschmerzen, persistierenten Gelenkschmerzen und Fibromyalgie.

4. Verwendung gemäß Anspruch 2 zur Prophylaxe und Behandlung von Schmerzen beim prämenstruellen Syndrom, Mastalgie, Bauchschmerzen beim Reizdarm und Schmerzen beim Carzinoid Syndrom.

5. Verwendung gemäß Anspruch 1 zur Prophylaxe und Behandlung von Kopfschmerzen und Migräne.

6. Verwendung gemäß Anspruch 1 zur Behandlung von Neuralgien, Trigeminusneuralgie, postherpetischen Neuralgien, neuropathischen Schmerzen und Tumor-assozüerten Schmerzen.

7. Verwendung gemäß eines der Ansprüche 1 - 6 in Kombination mit einem Opiat Analgetikum, Dihydrocodein, Tramadol, Morphin, Morphin-Sulfat, Oxycodon, Methadon, Hydromorphon, Buprenorphin oder Fentanyl.

8. Pharmazeutische Zusammensetzung enthaltend Tergurid oder Protergurid oder eine Kombination aus Tergurid und Protergurid oder deren pharmakologisch verträgliche Salze zusammen mit zumindest einem pharmakologisch verträglichen Träger, Hilfsstoff oder Lösungsmittel.

9. Pharmazeutische Zusammensetzung gemäß Anspruch 8 geeignet zur Inhalation oder zur intravenösen, intraperitonealen, intramuskulären, intravaginalen, intrabuccalen, perkutanen, subkutanen, mucokutanen, oralen, peroralen, lumbalen, rektalen, transdermalen, topikalen, intradermalen, intragastralen oder intrakutanen Applikation.

10. Pharmazeutische Zusammensetzung gemäß Anspruch 8 oder 9 des weiteren enthaltend einen Wirkstoff ausgewählt aus der Gruppe umfassend Opiat Analgetikum, Dihydrocodein, Tramadol, Morphin, Morphin-Sulfat, Oxycodon, Methadon, Hydromorphon, Buprenorphin und Fentanyl.

11. Pharmazeutische Zusammensetzung gemäß eines der Ansprüche 8 - 10 enthaltend Tergurid im Dosisbereich von 0,1 - 3,0 mg pro pharmazeutischer Formulierung und/oder Protergurid im Dosisbereich von 0,002 - 0,5 mg pro pharmazeutischer Formulierung.
